# EUROPEAN PATENT SPECIFICATION

(11) **EP 2 004 050 B1**
(45) Date of publication and mention of the grant of the patent: **03.11.2010**
(21) Application number: 07735325.8
(22) Date of filing: 29.03.2007
(51) Int. Cl.: A61B 5/103

(54) **METHOD AND APPARATUS FOR DETERMINING HYDRATION LEVELS FROM SKIN TURGOR**
VERFAHREN UND VORRICHTUNG ZUR BESTIMMUNG DES HYDRATATIONSGRADES VON HAUTSCHWELLUNGEN
METHODE ET APPAREIL POUR LA DETERMINATION DE NIVEAUX D'HYDRATATION A PARTIR D'UNE TURGESCENCE CUTANEE

(30) Priority: 31.03.2006 US 788455 P
(43) Date of publication of application: 24.12.2008
(73) Proprietor: Koninklijke Philips Electronics N.V., 5621 BA Eindhoven (NL)
(72) Inventor: COHEN-SOLAL, Eric, Briarcliff Manor, New York 10510-8001 (US); SHI, Yan S., Briarcliff Manor, New York 10510-8001 (US); RAJU, Balasundar, Briarcliff Manor, New York 10510-8001 (US)
(74) Representative: Van Velzen, Maaike Mathilde
(86) International application number: PCT/IB2007/051133
(87) International publication number: WO 2007/113755

(56) References cited:
- DE-A1- 3 304 503
- FR-A1- 2 707 112
- DOBREV HRISTO: "Use of Cutometer to assess epidermal hydration" SKIN RESEARCH AND TECHNOLOGY, vol. 6, 2000, pages 239-244, XP002445907
- HENDRIKS F.M. ET AL.: "A numerical-experimental method to characterize the non-linear mechanical behaviour of human skin" SKIN RESEARCH AND TECHNOLOGY, vol. 9, no. 3, August 2003 (2003-08), pages 274-283, XP002445908
- HENDRIKS F.M. ET AL.: "Influence of hydration and experimental length scale on the mechanical response of human skin in vivo, using optical coherence tomography" SKIN RESEARCH AND TECHNOLOGY, vol. 10, no. 4, November 2004 (2004-11), pages 231-241, XP002445909

## Description

Human beings and many animals rely on water to live. Water is essential to many biological and biochemical reactions that take place. As a result it is important maintain a minimum amount of water in the body.

Water is exchanged dynamically between the body and the environment. Under normal conditions, body fluids are well maintained in terms of both electrolyte concentrations and volume through processes like drinking, urine production and sweating. However, fluid balance may be disturbed due to a variety of reasons, including, but not limited to: insufficient water intake due to conditions such as chronic hypodipsia; gastrointestinal losses due to illness; renal conditions; skin losses; and clinical procedures such as hemodialysis.

All of these conditions can result in excessive fluid loss, and attendant dehydration or fluid deficit. More generally, dehydration refers to water loss with or without accompanied electrolyte loss, particularly sodium. Fluid loss of only a few percent of body weight causes discomfort and impaired body function. As dehydration levels increase, patients become fatigued and irritable, with symptoms of dry mouth, less-frequent urination and tachycardia.

Without proper water replenishment, fluid deficits can eventually develop to a clinical emergency when fluid loss is greater than 9% of body weight. Fluid deficits of such magnitudes can result in organ damage, coma, or even death.

From the above, it can be appreciated that the early identification of dehydration followed by prompt and adequate fluid intake can substantially reduce the risk of severe dehydration, and the potentially severe complications thereof.

Commonly, the clinical assessment of the level of hydration is mainly based on physical examination. Symptoms of dehydration include dry mouth and mucous membrane, sunken eyes, orthostatic hypotension, delayed capillary refill, and poor skin turgor. These symptoms are often recognized by physical examination. However, the clinical assessments can be subjective and have a low sensitivity and specificity in general.

Laboratory tests on blood and urine samples have also been used to determine dehydration status. Typically, laboratory tests are performed after physical assessment of dehydration symptoms to generate additional information; to validate the diagnosis; and to aid treatment. The main advantage of these tests over physical examination is that they provide objective and quantitative measurements. Nevertheless, they require special lab equipment and are usually time-consuming and costly.

There is a need, therefore, for a method and apparatus adapted to provide an accurate measure of hydration levels in patients that overcome at least some of the shortcomings described above. From DOBREV HRISTO ("Use of Cutometer to assess epidermal hydration" SKIN RESEARCH AND TECHNOLOGY, vol. 6, 2000, pages 239-244) is known a method for determing skin displacement parameters during hydration studies.

In accordance with an example embodiment, an apparatus, comprises a container disposed over a region of skin. The apparatus also includes a source of negative pressure connected to the container. A transducer is disposed in the container and is operative to transmit mechanical waves and to receive reflections of the mechanical waves. The apparatus also includes a receiver operative to receive electrical signals corresponding to the reflections and a processor operative to calculate a distance between the transducer and the region of skin over time. The distance over time is representative skin turgor.

In accordance with another example embodiment, a method, includes: applying a negative pressure to a region of skin; transmitting mechanical waves to the region of skin; receiving reflections of the mechanical waves from the region of the skin; calculating a distance from the transducer to the region of skin over time; and determining a hydration level of the body.

In accordance with yet another example embodiment, an apparatus includes a dehydration sensor. The dehydration sensor includes: a container disposed over a region of skin; a source of negative pressure connected to the container; and a plurality of transducers, each of which is operative to transmit mechanical waves and to receive reflections of the mechanical waves. The apparatus also includes a receiver operative to receive electrical signals corresponding to the reflections; and a processor operative to calculate a distance between the transducer and the region of skin over time, wherein the distance over time is representative skin turgor.

As used herein, the terms 'a' and 'an' mean one or more; and the term 'plurality' means two or more.

As used herein, the term 'patient' includes humans, mammals and fish.

The present teachings are best understood from the following detailed description when read with the accompanying drawing figures. It is emphasized that the various features are not necessarily drawn to scale. In fact, the dimensions may be arbitrarily increased or decreased for clarity of discussion.
Fig. 1 is a cross-sectional view of an apparatus in accordance with an example embodiment.
Fig. 2 is a simplified block diagram an apparatus in accordance with an example embodiment.
Fig. 3 is a cross-sectional view of an apparatus in accordance with another example embodiment.
Fig. 5 is a graphical representation of measurements of distance versus time in accordance with an example embodiment.

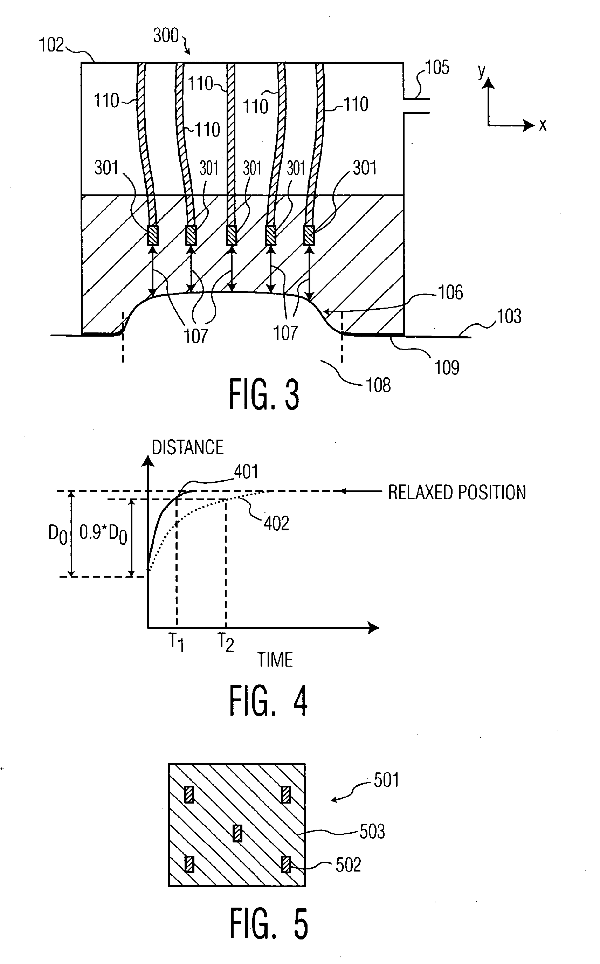

Fig. 5 is a top-view of a dehydration sensor in accordance with another example embodiment.

In the following detailed description, for purposes of explanation and not limitation, example embodiments disclosing specific details are set forth in order to provide a thorough understanding of the present teachings. However, it will be apparent to one having ordinary skill in the art having had the benefit of the present disclosure that other embodiments that depart from the specific details disclosed herein are contemplated. Moreover, descriptions of well-known devices, hardware, software, firmware, methods and systems may be omitted so as to avoid obscuring the description of the example embodiments. Nonetheless, such hardware, software, firmware, devices, methods and systems that are within the purview of one of ordinary skill in the art may be used in accordance with the example embodiments. Finally, wherever practical, like reference numerals refer to like features.

The example embodiments described relate primarily to A-scans. A-scan refers to a measurement technique where a transducer transmits mechanical waves at a surface of or into an object and the amplitudes of the reflected mechanical waves are recorded as a function of time. Normally, the transducer is located on one surface of the object and only structures that lie along the direction of propagation of the mechanical waves are interrogated. As reflected waves (echoes) return from interfaces within the object or tissue, the transducer converts the mechanical wave into a voltage that is proportional to the echo intensity.

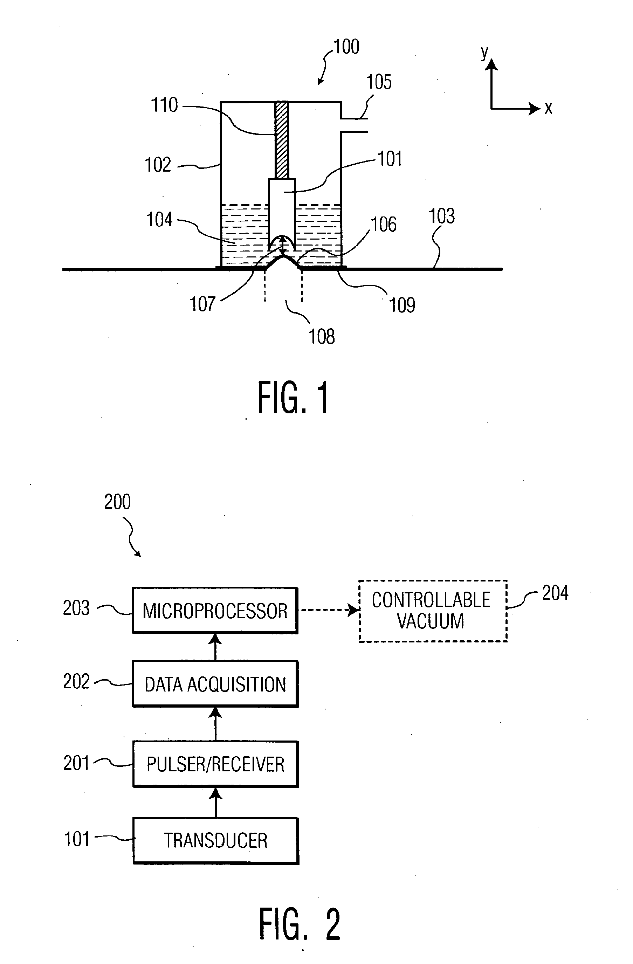

Fig. 1 is a cross-sectional view of an apparatus 100 in accordance with an example embodiment. The apparatus 100 includes a transducer 101 and electronics (not shown in Fig. 1). The transducer 101 is illustratively an ultrasonic transducer that emits mechanical waves having a frequency illustratively in the range of approximately 1.0 MHz to approximately 20.0 MHz. In a specific embodiment, the transducer is unfocused. Alternatively, the transducer 101 is a focused transducer.

In an illustrative embodiment, the transducer 101 is a single element transducer that operates in a pulsed mode. Accordingly, a relatively wide bandwidth, greater than approximately 25% is desirable. In addition, the frequency of the transducer is usefully relatively high to foster measurement accuracy. In a specific embodiment, the transducer 101 is adapted to operate in the range of approximately 10.0 MHz to approximately 20.0 MHz. In an alternative embodiment, a relatively low frequency transducer can be implemented as transducer 101.

The transducer 101 may be one of a variety of types of transducers useful in medical applications and known to those skilled in the art. For example, the transducer 101 may be a piezoelectric element such as a lead zirconate titanate (PZT) element. By way of another example, the transducer may be a piezoelectric micromachined ultrasonic transducer (PMUT). It is emphasized that the PZT and PMUT transducers are merely illustrative and that other transducers are contemplated.

Furthermore, in the interest of simplicity of description the transducer 101 performs both the transmit and the receive functions. It is contemplated that each apparatus includes a pair of transducers with one transducer performing the transmission function and the other performing the receive function. This variation is contemplated for the multiple transducer embodiments described herein, so that a pair of transducers in place of each of the transmit/receive transducers described. The electronic components and configuration of Fig. 2 may be embodied in a manner other than described expressly in connection with Fig. 2 to accommodate the transmit/receive transducer pairs. The requisite variations in the electronic components/configurations are within the purview of one of ordinary skill in the art.

The transducer 101 is disposed in a container 102 that is in contact with a stratum corneum layer 103 of the patient. A known coupling material 104 (e.g., water, air or a coupling gel) may be applied to the layer 103. The transducer 101 is immersed in the material 104 at least at the location of emission of the mechanical waves. As shown, the transducer 101 may be connected to the container by a structure 110, that provides support for the transducer 101 and includes the electrical connections between the transducer 101 and electrical components described in connection with Fig. 2.

The container 102 includes a vacuum connection 105. The vacuum connection may be connection to a known vacuum or other negative pressure source. The vacuum is applied to the container 102, causing a region 106 of the layer 103 to rise in the +y-direction as shown.

In an example embodiment, the container 102 is a substantially cylindrical tube with a relatively small circular opening 108 at the portion contacting the layer 103. The opening 108 is usefully smaller than the tube diameter and may have an adjustable diameter. The container 102 has a lower portion 109 about the opening 108 that contacts the with the skin. Beneficially, the lower portion 109 is relatively smooth and flat to insure good contact throughout the process without leaking of the coupling material 104. In embodiments in which the coupling material 104 is a liquid, a slight negative pressure (for example, approximately 5mbar) may be applied initially before placing the sensor on the skin, to prevent any leakage of the coupling material.

The apparatus 100 is useful in measuring skin turgor, which is the skin's propensity to return rapidly to its original contour after being raised. As is known, the greater the skin turgor, the faster the skin returns to its original contour, and the greater the hydration level of the patient. Notably, the dermis layer of the skin acts as a fluid (e.g., water) reservoir for the human body. As the body becomes dehydrated, the amount of fluid in the dermis is reduced. When the water/fluid content in the dermis decreases, the skin becomes less elastic and the skin turgor decreases. Thus, the measured skin turgor provides an indication of the level of dehydration.

In example embodiments, the patient may be a human being, and the apparatus 100 may be included in a medical testing device. It is contemplated that the apparatus 100 be used in veterinary testing of animals where concerns about dehydration require a measure of hydration levels.

In operation, the vacuum is activated raising the region 106 in the +y-direction. Mechanical waves are emitted from the transducer 101 and are reflected from the region 106 back onto the transducer. This provides an initial distance measurement of the distance 107 between the region 106 and the transducer 101. After the initial displacement is determined, the vacuum is released and the region 106 collapses in the -y-direction and ultimately returns to it original contour. At this point, skin turgor measurements are carried out in accordance with example embodiments.

In an illustrative embodiment the vacuum is released in a rapid fashion so the pressure in the container reaches the ambient pressure rather quickly. In another illustrative embodiment, the vacuum is released more slowly to control pressure equalization. As will be appreciated, the determination of the velocity of the region 106 (and thus the skin turgor) must be effected consistently. As such, the selected release of vacuum must be done consistently in both baseline measurements and specific measurements of skin turgor.

During the movement of region 106 of the skin 103 in the -y-direction to it original contour, the transducer 101 emits pulses of mechanical waves at periodic intervals. In a specific embodiment the periodicity of the intervals is approximately 2.0 ms or less (i.e., at a frequency of approximately 500 MHz or greater). These pulses are reflected at the region 106 of the layer 103 and are received at the transducer 101. The echoes or reflected waves are used to determine the time varying distance 107 between the region 106 and the transducer 101 as the region 106 moves in the -y-direction.

As described more fully herein, the distance versus time measurements are compared to a baseline for the population, or baseline levels for the patient, or both, and at acceptable hydration levels. If the distance versus time data is below the baseline, the skin turgor is lower than the baseline, and the patient is experiencing dehydration. In an example embodiment one value is calculated from the distance versus time measurement curve for comparison. The time value is determined from the distance versus time curve and the selected time value is compared to other time values. For example, in an embodiment the time required for the region 106 to travel 90% of its initial displacement is determined. This value is compared to the time required for region 106 to travel 90% of its initial displacement in a hydrated patient, for example. From these comparisons, the level of hydration of the patient can be determined.

In the present example embodiment, the mechanical waves are reflected from at the surface of the region 106 of the skin 103. The reflected waves (or echoes) are incident on the transducer 101. The mechanical waves are then converted into electrical signals by the transducer 101 and processed as described more fully herein.

The distance values for each emitted pulse/echo are calculated simply by multiplying the velocity of the mechanical waves in material 104 by the time between the transmission of a mechanical wave (or pulse) from the transducer and the reception of the reflected mechanical wave. Notably, the speed of mechanical waves in the material 104 is substantially constant, being substantially independent of the frequency of the mechanical wave and the temperature of the material 104. Accordingly, the distance from the transducer 101 to the region 106 can be calculated using the estimated constant velocity.

In accordance with an example embodiment, a plurality of measurements is made of the time varying distance 107. In another example embodiment, one or more measurements are made at each of a plurality of locations. To this end, the apparatus 100 may be applied to a number of locations on the body to garner skin turgor measurements at each of these locations. The measurements of distance 107 versus time between region 106 and the transducer 101 are garnered and compared to baseline values for the population, or the patient, or both to determine skin turgor and thus hydration levels. The measurement method at each location is substantially identical to that described previously. It is noted that a substantially identical value of vacuum or negative pressure is applied to the skin 103.Page: 10

Alternatively, a preset displacement of the region from the relaxed position is used. The vacuum pressure is varied using a controllable vacuum 204. The microprocessor 203 garners ultrasonic measurements of the displacement to actively control the vacuum pressure at the controllable vacuum 204 via a negative feedback loop. This process continues until the preset displacement is achieved. After the displacement is realized, the vacuum is released and the measurements of distance versus time are made.

Fig. 2 is a simplified schematic block diagram of an apparatus 200 in accordance with an example embodiment. Many features of the apparatus 200 are common to those of the apparatus described in connection with the example embodiments of Fig. 1. These common details may not be repeated in order to avoid obscuring the description of the present example embodiments.

The transducer 101 is connected to a pulse generator and receiver (PGR) 201. The PGR 201 transmits electrical pulses of finite duration and at a chosen periodicity to the transducer 101. The electrical pulses are converted to mechanical pulses that are emitted by the transducer 101 into material 104.

The emitted mechanical waves are reflected by the skin 106 and are received at the transducer 101. The reflected mechanical waves (echos) are converted into electrical signals by the transducer 101 and transmitted to the PGR 201. The PGR 201 includes a receiver circuit, filters and amplifiers. The amplifiers are useful particularly when the mechanical waves are relatively high frequency, as the attenuation of the mechanical waves in the material 104 increases with increasing frequency. Furthermore, the amplifiers may be useful to compensate for power in the mechanical waves dissipated by absorption in the skin 103 and diffusion of the waves. Suitable receiver circuits, filter circuits and amplifier circuits are well known to those skilled in analog signal processing.

The apparatus 200 also includes a data acquisition module 202. The module 202 illustratively includes a register or memory adapted to store received signal data from received from the PGR 202. For example, the module 202 may be an engine that calculates the time of flight of the transmitted mechanical wave for each transmitted pulse. These data are then stored for calculating the distance 107.

A processor/microprocessor 203 is provided to effect various functions of the apparatus 200. The microprocessor 203 may be a commercially available microprocessor such as a Pentium® from Intel Corporation, or another suitable processor. The processor 203 optionally includes operating system (OS) software. The processor 203 includes application code written to effect the algorithms described herein. Such code is within the purview of one of ordinary skill in the art.

In an example embodiment, the processor 203 is adapted to implement data capture and to carry out a correlation algorithm. Illustratively, the data capture includes analog to digital (A/D) conversion of received electrical signals, and storage of the data. Illustratively, the time of flight is determined based on a correlation algorithm.

Alternatively, the time of flight may be determined via edge detection, such as positive/negative slope, zero-crossing techniques known in the art. Notably, it is beneficial to perform a calibration sequence against a known distance using the selected algorithm. This ensures greater accuracy and consistency of the measurements.

It is emphasized that the noted algorithms are known to those skilled in the art and that other algorithms for determining the time-of-flight and distance measurements are contemplated.

In operation, upon receiving an input from an operator, the processor 203 triggers the transmission of pulses by the PGR 202 to begin a test. The processor 203 algorithmically determines the distance 107 at selected points in time by retrieving the time of flight of a pulse and multiplying the time by the velocity. The processor 203 may also determine an average distance versus time from a plurality of measurements at the same location; or an average distance versus time from a plurality of measurements from each of a plurality of locations.

Illustratively, many measurements from transmitted and reflected pulses may be effected at the same location and from these an average of the distance versus time may be determined. In a specific embodiment, more than five measurements are used to calculate an average.

Alternatively, pulses may be transmitted at more than one location (e.g., the forehead, the sternum, the abdominal region and the tibia) and the distance versus time determined for each of these locations. Multiple measurements can be made and an average distance versus time may be calculated. In a specific embodiment, more than five measurements at each location are used to calculate an average for each of the locations. The average values may also be stored in the data acquisition module 202.

After calculating the distance 107 versus time or an average distance over time as described above, the processor 203 applies algorithmically compares the distance 107 versus time or the average distance versus time, or both, of the most recent measurements with baseline turgor data. The algorithm may be a correlation algorithm adapted to correlate the measured data to baseline data that may be stored in a register or lookup table. From these comparisons, a relative measure of hydration levels can be made.

In accordance with an example embodiment, baseline turgor data may be garnered from multiple measurements performed on a patient. The data are used to compile a database useful in determining the relationship between skin turgor and dehydration levels at specific location for each patient. As alluded to previously, these measurements provide a baseline hydration value at acceptable (e.g., healthy) hydration levels that can then be used to map the measurements to the fluid hydration level in the patient. Alternatively or additionally, the baseline turgor values may be garnered from data from a large group of patients. These population-based baselines can be further compiled demographically so that a particular patient's hydration levels can be compared to acceptable fluid levels of people of similar height, weight, age and other similar criteria.

Fig. 3 is a cross-sectional view of an apparatus 300 in accordance with an example embodiment. Many features of the apparatus 300 are common to those of the apparati described in connection with the example embodiments of Figs. 1 and 2. These common details may not be repeated in order to avoid obscuring the description of the present example embodiments.

The apparatus includes a plurality of transducers 301. The transducers 301 are substantially identical to transducer 101 and may be coupled to the PRG 201 via a multiplexer (not shown) or through a PRG 201 having multiple inputs. The data from the transducers are individually processed as described previously.

Each transducer 301 is disposed in the material 104 and transmits mechanical waves to the region 106. The mechanical waves are reflected and each respective transducer 301 converts the echo into an electrical signal for processing in a substantially identical manner described. After the processor 103 performs a correlation algorithm comparing the acquired data to baseline values, the hydration levels of a patient may be determined.

In the present embodiment, each transducer 301 measures a respective distance 107. The measurements include the transmission, reflection and reception of mechanical waves as described previously. From measured time of flight of each pulse, the distance 107 versus time may be determined at each transducer location by the processor 203.

Optionally, a plurality of measurements may be made and an average fit compiled at the processor. In addition, the plurality of transducers 301 may be used at multiple locations and, optionally, a plurality of measurements may be made at each location.

The use of multiple transducers 301 provides greater levels of accuracy as multiple measurements are made in a localized area. This accuracy can be improved further by making multiple measurements.

Fig. 4 is a graphical representation showing the distance versus time in turgor measurements in accordance with an example embodiment. The skin of the patient has a rest position, which is the position of the skin without vacuum applied. Naturally, this displacement of the skin at rest over time remains nullity. The skin (e.g., region 106) is then displaced by an amount Do and released to garner the turgor measurements.

Curve 401 shows the displacement over time from an initial displacement Do of the patient at normal or acceptable hydration levels. Notably, this curve may be patient specific, or may be a composite value of population based on age, gender and other useful factors.

Curve 402 shows the displacement of the skin over time from an initial displacement Do of a patient. Curve 402 may be the curve for a maximum or threshold level of dehydration, for example. Alternatively, curve 402 may be the data of a patient not necessarily at a dangerous level of dehydration.

The illustrative method includes comparing the time for the skin to return from the initial displacement Do to predetermined fraction of that displacement. For example, in a specific embodiment shown in Fig. 4, the skin is displaced by Do and the time to return 90% of the displacement or 0.90 D₀. In this manner, when the skin returns to 0.10 Dₒ the time (T₂ in Fig. 4) is recorded and compared to the time (T₁ in Fig. 4) needed to return to 0.10 Dₒ in a normally hydrated patient.

These data are stored in the data acquisition module 202, retrieved from the module 202 by the microprocessor 203 and compared algorithmically in the microprocessor 203.

Fig. 5 is a top view of a dehydration sensor 501 in accordance with an example embodiment. The sensor 501 includes many features common to those of the apparati described in connection with the example embodiments of Figs. 1-4. These common details may not be repeated in order to avoid obscuring the description of the present example embodiments.

The sensor 501 includes a plurality of transducers 502 arranged in a substrate 503. In an example embodiment, the transducers 502 are substantially the same as the transducers 101, 303 described previously. The substrate 503 may be one of a variety of known materials that prevents mechanical and electrical interference between the transducers 502.Page: 16 Beneficially, the substrate 503 is a layer that assures substantially uniform contact between the transducers 502 and the skin. The substrate 503 should be substantially acoustically transparent (i.e., have a similar acoustic impedance as the skin) and relatively thin.

The sensor 501 is adapted to provide a plurality of measurements over a region of the body in a manner similar to that described in connection with the example embodiments of Figs. 3 and 4. The transducers 502 of the sensor 501 may be coupled to the PRG 201 via a multiplexer (not shown) or through a PRG 201 having multiple inputs. The data from the transducers are individually processed as described previously.

Each transducer 502 rests in a material such as material 104 useful in improving coupling of the transducer. The mechanical waves are reflected from the region 106 and are incident on respective transducers 502. The skin turgor measurements described previously are then carried out in a substantially identical manner to those previously described.

In a specific embodiment, each transducer 502 measures a respective distance 107 over the time that the region returns to its original contour. The measurements include the transmission, reflection and reception of mechanical waves as described previously. From measured time of flight the distance 107 over time may be determined at each transducer location by the processor 203.

The sensor 501 may also garner measurements from a number of locations on the body as described. Moreover, a plurality of measurements may be made at each location and an average for each calculated to garner the total average as described previously.

The measurement data stored in the module 202 can be compared to the baseline value for a determination of the level of dehydration in the patient. In an example embodiment, the measurement data from multiple locations may be used to determine the dehydration level. Moreover, average distance measurements described previously may be used to determine the dehydration level.

In view of this disclosure it is noted that the various methods and devices described herein can be implemented in hardware and software. Further, the various methods and parameters are included by way of example only and not in any limiting sense. In view of this disclosure, those skilled in the art can implement the present teachings in determining their own techniques and needed equipment to effect these techniques, while remaining within the scope of the appended claims.

## Claims

1. An apparatus, comprising:
a container (102) to be disposed over a region of skin;
a source of negative pressure connected to the container;
a transducer (101) disposed in the container and operative to transmit mechanical waves and to receive reflections of the mechanical waves;
a receiver (201) operative to receive electrical signals corresponding to the reflections;
a processor (203) operative to calculate a distance between the transducer and the region of skin over time, wherein the distance (107) over time is representative of skin turgor.

2. An apparatus as recited in claim 1, wherein the container and the source of negative pressure are adapted to raise the region of the skin.

3. An apparatus as recited in claim 2, wherein the processor is operative to garner a plurality of measurements from the transducer and to calculate a distance over time for each of the measurements.

4. An apparatus as recited in claim 3, wherein the processor is operative to compare the distance over time with a baseline distance versus time.

5. An apparatus as recited in claim 1, wherein the mechanical waves are ultrasonic waves.

6. An apparatus as recited in claim 3, wherein the plurality of calculations from the transducer are from more than one location on a body.

7. A method, comprising:
applying a negative pressure to a region of skin;
transmitting mechanical waves to the region of skin;
receiving reflections of the mechanical waves from the region of the skin;
calculating a distance from the transducer to the region of skin over time; and
determining a hydration level of the body from the distance over time

8. A method as recited in claim 7, wherein the calculating further comprises selecting an initial distance from the transducer to the region of the skin; measuring a first time for the region of skin to relax to a fraction of the initial distance; measuring a second time for the region of skin to relax to the fraction of the initial distance in a normally hydrated patient; and the determining further comprises comparing the first and second times.

9. A method as recited in claim 7, further comprising providing a plurality of transducers at an area where the mechanical waves are introduced into the body, and each of the transducers is operative to emit the mechanical waves and to receive the reflections.

10. A method as recited in claim 7, further comprising performing the transmitting and the receiving at least twice; calculating the distance from the transducer to the region of skin over time for each transmission and reception; and correlating the distances over time to baseline distances over time.

11. A method as recited in claim 10, wherein the determining the hydration level further comprises calculating an average distance over time and correlating the average distance over time to an average baseline distance over time.

12. A method as recited in claim 7, further comprising performing the transmitting and receiving at a plurality of locations on the body.

13. A method as recited in claim 12, further comprising:
calculating an average distance over time for each location, wherein the determining the hydration level further comprises comparing the average distance for each location with a baseline distance for each location.

14. An apparatus, comprising:
a dehydration sensor comprising:
a container (102) to be disposed over a region of skin;
a source of negative pressure connected to the container; and a plurality of transducers (301), each of which is operative to transmit mechanical waves and to receive reflections of the mechanical waves;
a receiver operative to receive electrical signals corresponding to the reflections; and
a processor (203) operative to calculate a distance between the transducer and the region of skin over time, wherein the distance (107) over time is representative of skin turgor.

15. An apparatus as recited in claim 14, wherein the container and the negative pressure source are adapted to raise the region of the skin.

16. An apparatus as recited in claim 15, wherein the processor is operative to garner a plurality of measurements from the transducer and to calculate a distance over time for each of the measurements.

17. An apparatus as recited in claim 16, wherein the processor is operative to compare the distance over time with a baseline distance versus time.

18. An apparatus as recited in claim 14, wherein the mechanical waves are ultrasonic waves.

19. An apparatus as recited in claim 3, wherein the plurality of calculations from the transducer are from more than one location on a body.

## Patentansprüche

1. Gerät, das Folgendes umfasst:
einen Behälter (102) zur Anordnung über einer Hautregion;
eine mit dem Behälter verbundene Quelle negativen Drucks;
einen in dem Behälter angeordneten Wandler (101), der wirksam ist, um mechanische Wellen auszusenden und Reflektionen der mechanischen Wellen zu empfangen;
einen Empfänger (201), der wirksam ist, um elektrische Signale zu empfangen, die den Reflexionen entsprechen;
einen Prozessor (203), der wirksam ist, um einen Abstand zwischen dem Wandler und der Hautregion über die Zeit zu berechnen, wobei der Abstand (107) über die Zeit für den Hautturgor repräsentativ ist.

2. Gerät nach Anspruch 1, wobei der Behälter und die Quelle negativen Drucks vorgesehen sind, um die Hautregion anzuheben.

3. Gerät nach Anspruch 2, wobei der Prozessor wirksam ist, um eine Vielzahl von Messungen von dem Wandler zu sammeln und einen Abstand über die Zeit für jede der Messungen zu berechnen.

4. Gerät nach Anspruch 3, wobei der Prozessor wirksam ist, um den Abstand über die Zeit mit einem Grundabstand gegenüber der Zeit zu vergleichen.

5. Gerät nach Anspruch 1, wobei die mechanischen Wellen Ultraschallwellen sind.

6. Gerät nach Anspruch 3, wobei die Vielzahl von Berechnungen von dem Wandler von mehr als einer Stelle auf einem Körper stammen.

7. Verfahren, das Folgendes umfasst:
Anwenden eines negativen Drucks auf eine Hautregion;
Aussenden mechanischer Wellen zu der Hautregion;
Empfangen von Reflexionen der mechanischen Wellen von der Hautregion;
Berechnen eines Abstands von dem Wandler zur Hautregion über die Zeit;
und
Bestimmen eines Hydratationsgrades des Körpers anhand des Abstands über die Zeit.

8. Verfahren nach Anspruch 7, wobei das Berechnen weiterhin Folgendes das umfasst: Auswählen eines ersten Abstands von dem Wandler zu der Hautregion; Messen einer ersten Zeit, die die Hautregion zum Entspannen auf einen Bruchteil des ersten Abstands benötigt; Messen einer zweiten Zeit, die die Hautregion zum Entspannen auf den Bruchteil des ersten Abstands bei einem normal hydrierten Patienten benötigt; und wobei das Bestimmen das Vergleichen der ersten und der zweiten Zeit umfasst.

9. Verfahren nach Anspruch 7, das weiterhin das Schaffen einer Vielzahl von Wandlern bei einem Bereich umfasst, wo mechanische Wellen in den Körper eingeführt werden, und wobei jeder der Wandler wirksam ist, um die mechanischen Wellen zu emittieren und die Reflexionen zu empfangen.

10. Verfahren nach Anspruch 7, das weiterhin Folgendes umfasst: Durchführen des Aussendens und des Empfangens mindestens zwei Mal; Berechnen des Abstands vom Wandler zu der Hautregion über die Zeit für jedes Aussenden und Empfangen; und Korrelieren der Abstände über die Zeit mit Grundabständen über die Zeit.

11. Verfahren nach Anspruch 10, wobei das Bestimmen des Hydratationsgrades weiterhin das Berechnen eines durchschnittlichen Abstands über die Zeit und das Korrelieren des durchschnittlichen Abstands über die Zeit mit einem durchschnittlichen Grundabstand über die Zeit umfasst.

12. Verfahren nach Anspruch 7, das weiterhin das Durchführen des Aussendens und Empfangens an einer Vielzahl von Stellen auf dem Körper umfasst.

13. Verfahren nach Anspruch 12, das weiterhin Folgendes umfasst:
Berechnen eines durchschnittlichen Abstands über die Zeit für jede Stelle, wobei das Bestimmen des Hydratationsgrades weiterhin das Vergleichen des durchschnittlichen Abstands für jede Stelle mit einem Grundabstand für jede Stelle umfasst.

14. Gerät, das Folgendes umfasst:
einen Dehydratationssensor, der folgendes umfasst:
einen Behälter (102) zur Anordnung über einer Hautregion;
eine mit dem Behälter verbundene Quelle negativen Drucks; und eine Vielzahl von Wandlern (301), die jeweils wirksam sind, um mechanische Wellen auszusenden und Reflektionen der mechanischen Wellen zu empfangen;
einen Empfänger, der wirksam ist, um elektrische Signale zu empfangen, die den Reflexionen entsprechen; und
einen Prozessor (203), der wirksam ist, um einen Abstand zwischen dem Wandler und der Hautregion über die Zeit zu berechnen, wobei der Abstand (107) über die Zeit für den Hautturgor repräsentativ ist.

15. Gerät nach Anspruch 14, wobei der Behälter und die Quelle negativen Drucks vorgesehen sind, um die Hautregion anzuheben.

16. Gerät nach Anspruch 15, wobei der Prozessor wirksam ist, um eine Vielzahl von Messungen von dem Wandler zu sammeln und einen Abstand über die Zeit für jede der Messungen zu berechnen.

17. Gerät nach Anspruch 16, wobei der Prozessor wirksam ist, um den Abstand über die Zeit mit einem Grundabstand gegenüber der Zeit zu vergleichen.

18. Gerät nach Anspruch 14, wobei die mechanischen Wellen Ultraschallwellen sind.

19. Gerät nach Anspruch 3, wobei die Vielzahl von Berechnungen von dem Wandler von mehr als einer Stelle auf einem Körper stammen.

## Revendications

1. Appareil, comprenant :
un conteneur (102) destiné à être disposé au-dessus d'une région de peau ;
une source de pression négative reliée au conteneur ;
un transducteur (101) disposé dans le conteneur et opérationnel pour transmettre des ondes mécaniques et pour recevoir des réflexions des ondes mécaniques ;
un récepteur (201) opérationnel pour recevoir des signaux électriques correspondant aux réflexions ;
un processeur (203) opérationnel pour calculer une distance entre le transducteur et la région de peau au fil du temps, dans lequel la distance (107) au fil du temps est représentative d'une turgescence cutanée.

2. Appareil selon la revendication 1, dans lequel le conteneur et la source de pression négative sont adaptés pour lever la région de la peau.

3. Appareil selon la revendication 2, dans lequel le processeur est opérationnel pour collecter une pluralité de mesures du transducteur et pour calculer une distance au fil du temps pour chacune des mesures.

4. Appareil selon la revendication 3, dans lequel le processeur est opérationnel pour comparer la distance au fil du temps avec une distance en fonction du temps de ligne de base.

5. Appareil selon la revendication 1, dans lequel les ondes mécaniques sont des ondes ultrasoniques.

6. Appareil selon la revendication 3, dans lequel la pluralité de calculs par le transducteur provient de plus d'un emplacement sur un corps.

7. Procédé comprenant :
l'application d'une pression négative sur une région de peau ;
la transmission d'ondes mécaniques à la région de peau ;
la réception de réflexions des ondes mécaniques depuis la région de la peau ;
le calcul d'une distance du transducteur à la région de peau au fil du temps ;
et
la détermination d'un niveau d'hydratation du corps, à partir de la distance au fil du temps.

8. Procédé selon la revendication 7, dans lequel le calcul comprend en outre le choix d'une distance initiale du transducteur à la région de la peau ; la mesure d'une première fois où la région de peau se relaxe à une fraction de la distance initiale ; la mesure d'une deuxième fois où la région de peau se relaxe à la fraction de la distance initiale chez un patient normalement hydraté ; et la détermination comprend en outre une comparaison des première et deuxième fois.

9. Procédé selon la revendication 7, comprenant en outre la fourniture d'une pluralité de transducteurs au niveau d'une zone où les ondes mécaniques sont introduites dans le corps, et chacun des transducteurs est opérationnel pour diffuser les ondes mécaniques et pour recevoir les réflexions.

10. Procédé selon la revendication 7, comprenant en outre l'exécution de la transmission et de la réception au moins deux fois ; un calcul de la distance du transducteur à la région de peau au fil du temps pour chaque transmission et réception ; et une corrélation des distances au fil du temps aux distances au fil du temps de ligne de base.

11. Procédé selon la revendication 10, dans lequel la détermination du niveau d'hydratation comprend en outre le calcul d'une distance moyenne au fil du temps et une corrélation de la distance moyenne au fil du temps à une distance moyenne au fil du temps de ligne de base.

12. Procédé selon la revendication 7, comprenant en outre l'exécution de la transmission et de la réception au niveau d'une pluralité d'emplacements sur le corps.

13. Procédé selon la revendication 12, comprenant en outre :
le calcul d'une distance moyenne au fil du temps pour chaque emplacement, dans lequel la détermination du niveau d'hydratation comprend en outre une comparaison de la distance moyenne pour chaque emplacement avec une distance de ligne de base pour chaque emplacement.

14. Appareil, comprenant :
un capteur de déshydratation comprenant :
un conteneur (102) destiné à être disposé au-dessus d'une région de peau ;
une source de pression négative reliée au conteneur ; et une pluralité de transducteurs (308), dont chacun est opérationnel pour transmettre des ondes mécaniques et pour recevoir des réflexions des ondes mécaniques ;
un récepteur opérationnel pour recevoir des signaux électriques correspondant aux réflexions ; et
un processeur (203) opérationnel pour calculer une distance entre le transducteur et la région de peau au fil du temps, dans lequel la distance (107) au fil du temps est représentative d'une turgescence cutanée.

15. Appareil selon la revendication 14, dans lequel le conteneur et la source de pression négative sont adaptés pour lever la région de la peau.

16. Appareil selon la revendication 15, dans lequel le processeur est opérationnel pour collecter une pluralité de mesures du transducteur et pour calculer une distance au fil du temps pour chacune des mesures.

17. Appareil selon la revendication 16, dans lequel le processeur est opérationnel pour comparer la distance au fil du temps avec une distance en fonction du temps de ligne de base.

18. Appareil selon la revendication 14, dans lequel les ondes mécaniques sont des ondes ultrasoniques.

19. Appareil selon la revendication 3, dans lequel la pluralité de calculs par le transducteur provient de plus d'un emplacement sur un corps.
